# EUROPEAN PATENT APPLICATION

(11) **EP 4 159 234 A1**
(43) Date of publication of application: **05.04.2023**
(21) Application number: 21818884.5
(22) Date of filing: 01.04.2021
(51) Int. Cl.: A61K 39/215, C12N 15/861, A61P 31/14

(54) **SARS-COV-2 VACCINE**

(30) Priority: 01.06.2020 CN 202010485668; 24.08.2020 CN 202010866719
(71) Applicant: Cansino Biologics Inc., Tianjin 300457 (CN); Academy of Military Medical Sciences, PLA, Beijing 100850 (CN)
(72) Inventor: LI, Junqiang, Tianjin 300457 (CN); SI, Weixue, Tianjin 300457 (CN); ZHU, Tao, Tianjin 300457 (CN); XU, Yunli, Tianjin 300457 (CN); DENG, Jie, Tianjin 300457 (CN); CHAO, Shoubai, Tianjin 300457 (CN)
(74) Representative: HGF
(86) International application number: PCT/CN2021/084806
(87) International publication number: WO 2021/244120

(57) **Abstract**

Disclosed is a SARS-CoV-2 vaccine, wherein the S protein of SARS-CoV-2 serves as the antigen, and the vaccine comprises an adenoviral vector, and the vaccine induces an improved protective immune response through mucosal immunity, thus preventing SARS-CoV-2 infection. Specifically, when atomized by an appropriate apparatus, the vaccine generates particles of improved uniformity, which can reach the lungs after being inhaled via the nasal cavity or the oral cavity, thus producing a protective immune response with respect to the entire respiratory tract and the lungs, enhancing the effective utilization rate of the vaccine, and increasing the effect of the vaccine.

## Description

### TECHNICAL FIELD

The invention relates to the technical field of vaccines, in particular to a SARS-CoV-2 vaccine, for which the S protein of SARS-CoV-2 serves as the antigen, the form of the vaccine includes but is not limited to an adenoviral vector vaccine, and the vaccine induces an improved protective immune response through mucosal immunity, thus preventing a SARS-CoV-2 infection.

### BACKGROUND

Coronavirus is a non-segmented single-stranded positive-sense RNA virus, belonging to the subfamily Orthocoronavirinae in the family Nidovirales in the order Coronaviridae. According to the serotype and genomic characteristics, the subfamily of coronavirus is divided into four genera of α, β, γ and δ. To date, there are 7 coronaviruses that can infect humans: 229E and NL63 of genera α, OC43 and HKU1 of genera β, Middle East Respiratory Syndrome-associated Coronavirus (MERSr CoV), Severe Acute Respiratory Syndrome-associated Coronavirus (SARSr CoV) and Novel Coronavirus (SARS-CoV-2).

The novel coronavirus (SARS-CoV-2) can cause acute respiratory distress syndrome, septic shock, bleeding and coagulation dysfunction, thus leading to novel coronavirus-associated diseases (COVID-19).

Viruses of the genus Coronavirus are enveloped positive-sense single-stranded RNA viruses, about 80-120 nm in diameter and 27-32 Kb in genome, whose genetic material is the largest of all the RNA viruses; the structural features of the positive-sense strand RNA of the genus Coronavirus are that a methylated "cap" is at the 5' end of the RNA strand and a PolyA "tail" structure is at the 3' end. This structure is very similar to that of eukaryotic mRNA, which is an important structural basis for coronavirus genomic RNA itself to act as a translation template. After infection of host cells by coronaviruses, proteins can be synthesized directly and RNA-DNA-RNA transcription is omitted. This feature contributes to the susceptibility of coronaviruses to mutation or to genetic recombination.

The coronavirus can infect vertebrates such as humans, mice, pigs, cats, dogs and birds. The coronavirus has an envelope, and there are spikes on the envelope. The whole virus is like the corona. The spikes of different coronaviruses are obviously different. Tubular inclusion bodies can sometimes be seen in cells infected with coronavirus.

The S protein is the most important surface protein of coronavirus, which is related to the infectivity of the virus. The S protein contains two subunits: S 1 and S2. S 1 mainly contains a receptor binding domain (RBD), which is responsible for recognizing cell receptors; S2 contains the basic elements required for the membrane fusion process. The S protein is responsible for the binding and membrane fusion between the virus and the host cell membrane receptor; the S protein determines the host range and specificity of the virus; the S protein can be transmitted between different hosts through gene recombination or mutation of the receptor binding domain (RBD), which leads to high mortality; the S protein can produce neutralizing antibodies, so the S protein is an important candidate antigen for vaccine design.

The S protein is the preferred antigen for the development of the coronavirus vaccine. A comprehensive analysis of relevant reports on SARS, MERS and SARS-COV-2 vaccine research is essentially designed around the S antigen. Five different technical routes have been laid out to develop the SARS-COV-2 vaccine in China, including the nucleic acid vaccine, the recombinant protein vaccine, the inactivated vaccine, the vector vaccine and the influenza virus vector vaccine. These vaccines are immunized by intramuscular injection. The novel coronavirus is a respiratory infection virus. The current research results show that the novel coronavirus accumulates in the lower respiratory tract and lungs, therefore, effective elimination of viruses in the respiratory system and lungs is a strategy that must be considered when developing the novel coronavirus vaccine.

### SUMMARY

The invention provides a SARS-CoV-2 vaccine, which uses a transmucosal drug delivery system and is a recombinant adenoviral vector vaccine. The recombinant adenovirus is inserted with the S protein gene of SARS-CoV-2.

Specifically, the above transmucosal drug delivery system is selected from: a nasal drop, an aerosol, a spray, a powder spray, a powder, gel, a microsphere agent, a liposome, a membrane, and a suspension, etc.

Specifically, according to the classification of the substance form, the above transmucosal drug delivery system can be of the liquid dosage form (such as a suspension, which can form an aerosol or a spray when administered by a specific device), the solid dosage form (such as a powder, which can form a powder spray when administered by a specific device), the semisolid dosage form (such as gel, a membrane), or the gas dosage form (such as an aerosol, a spray).

In one embodiment of the invention, the transmucosal drug delivery system is of the gas dosage form, such as an aerosol, a spray, etc.

Specifically, the recombinant adenovirus can also contain other structural protein (such as the M protein, E protein, N protein) genes (full or partial sequences) of SARS-CoV-2.

In one embodiment of the invention, the recombinant adenovirus comprises the S protein gene and M protein gene of SARS-CoV-2.

In one embodiment of the invention, the recombinant adenovirus comprises the S protein gene and E protein gene of SARS-CoV-2.

In one embodiment of the invention, the recombinant adenovirus comprises the S protein gene, M protein gene and E protein gene of SARS-CoV-2.

Specifically, the above adenovirus can be the human adenovirus (such as the AdHu2 type, AdHu5 type, etc.) and the animal adenoviral vector, such as the chimpanzee adenoviral vector (such as the AdC6 type, AdC7 type, AdC36 type, AdC68 type, etc.); in one embodiment of the invention, the adenovirus is the AdHu5 type.

In particular, in the above vaccine, the content of the recombinant adenovirus is between 1×10⁹~5×10¹¹VP/ml (in particular, such as 2×10⁹, 4×10⁹, 6×10⁹, 8×10⁹, 1 ×10¹⁰, 2×10¹⁰, 4×10¹⁰, 6×10¹⁰, 8×10¹⁰, 1×10¹¹, 2×10¹¹, 3×10¹¹, 4×10¹¹, and 5×10¹¹ VP/ml); in one embodiment of the invention, the content of the recombinant adenovirus is 1×10¹¹VP/ml.

Specifically, the vaccine also contains pharmaceutically acceptable auxiliary materials.

Specifically, the pharmaceutically acceptable auxiliary materials can be selected from one or more of a buffer, a protective agent, a stabilizer, a surfactant and an osmotic pressure regulator.

In one embodiment of the invention, the vaccine is of a liquid dosage form.

Specifically, in the above liquid dosage form, the content of the buffer can be 0-10mM (such as 1mM, 2mM, 3mM, 4mM, 5mM, 6mM, 7mM, 8mM, 9mM), especially 0-5mM.

In one embodiment of the invention, the buffer can include one or more of HEPES, HIS, TRIS, succinic acid and citric acid; in one embodiment of the invention, the buffer is HEPES. Specifically, the content of HEPES in the transmucosal drug delivery liquid dosage form can be 0-10mM, especially 1-5mM.

In one embodiment of the invention, the buffer can include His; Specifically, the content of His in the liquid dosage form can be 0-10mM, especially 3-7mM.

Specifically, the protective agent can include a freeze-dried protective agent, an antigen protective agent, etc.

Specifically, the antigen protective agent can be gelatin and/or human serum albumin. In one embodiment of the invention, the antigen protective agent comprises gelatin; specifically, the content of gelatin in the liquid dosage form is 0-20mg/ml (in particular, such as 1mg/ml, 2mg/ml, 3mg/ml, 4mg/ml, 5mg/ml, 6mg/ml, 7mg/ml, 8mg/ml, 9mg/ml, 10mg/ml, 11mg/ml, 12mg/ml, 13mg/ml, 14mg/ml, 15mg/ml, 16mg/ml, 17mg/ml, 18mg/ml, 19mg/ml), especially 5-15mg/ml.

In one embodiment of the invention, the antigen protective agent can include the human recombinant albumin (HSA); specifically, the content of HSA in the liquid dosage form is 0-10% (weight percentage) (specifically, such as 0.1%, 0.2%, 0.3%, 0.4%, 0.5%, 0.6%, 0.7%, 0.8%, 0.9%, 1%, 2%, 4%, 6%, 8%, 10%), especially 0.1% - 1%.

Specifically, in the above liquid dosage form, the content of the stabilizer can be 0-100mg/ml (in particular, such as 5mg/ml, 10mg/ml, 15mg/ml, 20mg/ml, 25mg/ml, 30mg/ml, 35mg/ml, 40mg/ml, 45mg/ml, 50mg/ml, 55mg/ml, 60mg/ml, 65mg/ml, 70mg/ml, 75mg/ml, 80mg/ml, 85mg/ml, 90mg/ml, 95mg/ml).

Specifically, in the liquid dosage form, the stabilizer can be one or more of sucrose, mannitol, fucose and maltose; the main function of the stabilizer is to maintain the activity of the virus during freeze-thawing, freeze-storage or freeze-drying of the liquid preparation.

In one embodiment of the invention, the stabilizer can include sucrose; specifically, the content of sucrose in the liquid dosage form can be 0-100mg/ml, especially 10-50mg/ml.

In one embodiment of the invention, the stabilizer can include mannitol; specifically, the content of mannitol in the liquid dosage form can be 0-100mg/ml, especially 15-75mg/ml. Specifically, in the above vaccine, the content of surfactant can be 0-10mg/ml (for example, 0.01mg/ml, 0.05mg/ml, 0.1mg/ml, 0.2mg/ml, 0.3mg/ml, 0.4mg/ml, 0.5mg/ml, 1mg/ml, 1.5mg/ml, 2mg/ml, 2.5mg/ml, 3mg/ml, 3.5mg/ml, 4mg/ml, 4.5mg/ml, 5mg/ml, 6mg/ml, 7mg/ml, 8mg/ml, 9mg/ml).

In one embodiment of the invention, the surfactant can include Tween (such as Tween 80); specifically, the content of Tween in the liquid dosage form is 0-10mg/ml, especially 0.05-0.5mg/ml.

In one embodiment of the invention, the surfactant can include glycerin; specifically, the content of glycerin in the liquid dosage form is 0-10mg/ml, especially 0.5-5mg/ml.

Specifically, the content of the osmotic pressure regulator in the above liquid dosage form can be 0-100mM (in particular, such as 1mM, 10mM, 15mM, 20mM, 25mM, 30mM, 35mM, 40mM, 45mM, 50mM, 55mM, 60mM, 65mM, 70mM, 75mM, 80mM, 85mM, 90mM, 95mM), especially 40-60mM.

In one embodiment of the invention, the osmotic pressure regulator comprises sodium chloride.

Specifically, the content of the antigen protector in the above liquid dosage form can be 0-10mM (in particular, such as 0.01mM, 0.05mM, 0.1mM, 0.2mM, 0.3mM, 0.4mM, 0.5mM, 1mM, 2mM, 3mM, 4mM, 5mM, 6mM, 7mM, 8mM, 9mM, 10mM).

In one embodiment of the invention, the virus active protective agent can include magnesium chloride; specifically, the content of magnesium chloride in the liquid dosage form is 0-10mM, especially 1-5mM.

In one embodiment of the invention, the virus active protective agent can include EDTA; specifically, the content of EDTAin the liquid dosage form is 0-10mM, especially 0.05-0.5MM.

In one embodiment of the invention, the vaccine comprises the recombinant adenovirus, sucrose, mannitol, sodium chloride, HEPES, magnesium chloride, Tween 80 and gelatin.

In one embodiment of the invention, the vaccine comprises the recombinant adenovirus, sucrose, mannitol, sodium chloride, glycerin, HEPES, magnesium chloride, Tween 80, His, EDTA, HSA and gelatin.

In one embodiment of the invention, the vaccine is of a liquid dosage form, which comprises: 1×10⁹~5×10¹¹VP/ml of a recombinant adenovirus, 10-50mg/ml of sucrose, 15-75mg/ml of mannitol, 40-60mM of sodium chloride, 1-5mM of HEPES, 1-5mM of magnesium chloride, 0.05-0.5mg/ml of Tween 80, and 5-15mg/ml of gelatin.

In one embodiment of the invention, the vaccine is of a liquid dosage form, which includes: 1×10⁹~5×10¹¹VP/ml of a recombinant adenovirus, 10-50mg/ml of sucrose, 15-75mg/ml of mannitol, 40-60mM of sodium chloride, 0.5-5mg/ml of glycerin, 1-5mm of HEPES, 1-5mm of magnesium chloride, 0.05-0.5mg/ml of Tween 80, 3-7mM of His, 0.05-0.5mM of EDTA, 0.1% - 1% of HSA, and 5-15mg/ml of gelatin.

In one embodiment of the invention, the vaccine is of a liquid dosage form, which includes: 1×10¹¹VP/ml of a recombinant adenovirus, 25mg/ml of sucrose, 50mg/ml of mannitol, 50mM of sodium chloride, 2.5 mM of HEPES, 2mM of magnesium chloride, 0.1mg/ml of Tween 80, and 10mg/ml of gelatin.

In one embodiment of the invention, the vaccine is a liquid dosage form, which includes: 1×10¹¹VP/ml of a recombinant adenovirus, 25mg/ml of sucrose, 50mg/ml of mannitol, 50mM of sodium chloride,1.5mg/ml of glycerin, 2.5 mM of HEPES, 2mM of magnesium chloride, 0.1mg/ml of Tween 80, 5mM of His, 0.1mM of EDTA, 0.60% of HSA, and 10mg/ml of gelatin.

In one embodiment of the invention, the vaccine of the invention is of a solid dosage form, which can be obtained by treating the liquid dosage form of the invention (such as freeze-drying).

In one embodiment of the invention, the vaccine of the invention is of a gas dosage form, which can be obtained from the liquid dosage form or solid dosage form of the invention after treatment (such as atomization).

Specifically, those skilled in the art can also add one or more of other auxiliary materials, such as a propellant, an absorption promoter, a preservative, a diluent, an excipient, a cosolvent, etc., into the vaccine according to actual needs.

The invention also provides a preparation method of the vaccine, which includes the preparation method steps of the recombinant adenovirus.

Specifically, any suitable recombinant adenovirus preparation method known in the prior art can be used for the above recombinant adenovirus preparation method, for example, any suitable available adenovirus packaging system and service in the prior art can be used (for example, but not limited to the adenovirus packaging system and adenovirus packaging service that can be provided by Wuhan Viraltherapy Technologies Co., Ltd., Wuhan Biofavor Biotechnology Service Co., Ltd., etc.).

Specifically, the preparation method can include the following steps:
(1) the recombinant adenovirus shuttle plasmid was obtained by connecting the S protein gene of SARS-CoV-2 with the shuttle plasmid of the adenovirus;
(2) packaging cells were co-transfected by the recombinant adenovirus shuttle plasmid obtained in step (1) and the skeleton plasmid carrying most of the adenovirus genome to obtain the recombinant adenovirus carrying the S protein gene of SARS-CoV-2.

In one embodiment of the invention, the packaging cells are 293 cells.

The invention also provides use of the S protein gene of SARS-CoV-2 in preparing of a vaccine to prevent a SARS-CoV-2 infection, in particular, the vaccine is a transmucosal drug delivery system.

The invention also provides use of a recombinant adenovirus in the preparation of a vaccine to prevent a SARS-CoV-2 infection, the recombinant adenovirus contains the S protein gene of SARS-CoV-2, in particular, the vaccine is a transmucosal drug delivery system.

Specifically, the adenovirus can be a human adenovirus (such as AdHu2 type, AdHu5 type, etc.), a chimpanzee adenoviral vector (such as AdC6 type, AdC7 type, AdC36 type, AdC68 type, etc.); in one embodiment of the invention, the adenovirus is of the AdHu5 type.

Specifically, in the above use, the transmucosal drug delivery system is selected from: a nasal drop, an aerosol, a spray, a powder spray, a powder, gel, a microsphere agent, a liposome, a membrane, a suspension, etc.

Specifically, according to the classification of the substance form, the above transmucosal drug delivery system can be of the liquid dosage form (such as a suspension, which can form an aerosol or a spray when administered by a specific device), the solid dosage form (such as a powder, which can form a powder spray when administered by a specific device), the semisolid dosage form (such as gel, a membrane), or the gas dosage form (such as an aerosol, a spray).

In one embodiment of the invention, the transmucosal drug delivery system is of the gas dosage form, such as an aerosol, a spray, a powder spray, etc.

The invention also provides a method for preventing COVID-19, which includes the step of administering an effective amount of the vaccine of the invention to a subject (especially by transmucosal administration).

In one embodiment of the invention, the transmucosal administration is atomized inhalation administration.

In another embodiment of the invention, the transmucosal administration is nasal drip administration.

Specifically, in the above method, a variety of transmucosal administration means can be used, such as nasal drip administration and aerosol inhalation administration for subjects; the vaccine of the invention can also be administered to the subject in combination with other administration means (such as injection, specifically intramuscular injection) to enhance the immune effect, such as aerosol inhalation administration and intramuscular injection, or nasal drip administration and intramuscular injection, or intramuscular injection, nasal drip administration and aerosol inhalation administration to the subject.

Specifically, in the above method, the transmucosal immune preparation is aerosolized to form particles of 10 µm or below, preferably in the range of 3-10 µm, and after inhalation through the oral or nasal cavity, can be uniformly distributed throughout the respiratory tract, including the lungs.

The invention also provides a drug delivery device, which comprises the vaccine of the invention.

Specifically, the above drug delivery device can include an atomizer, a spray, etc. The vaccine of the invention is of a liquid dosage form or a solid dosage form, which can form a gas dosage form through the drug delivery device, such as an aerosol, a spray, a powder spray, etc.

Specifically, in the above method, the device may aerosolize the vaccine to form particles of 10 µm or below, preferably in the range of 3-10 µm, and may be evenly distributed throughout the respiratory tract, including the lungs, after oral or nasal inhalation.

The invention provides a transmucosal immune SARS-CoV-2 vaccine, which takes the S protein of SARS-CoV-2 as the antigen, and can produce a better protective immune response after transmucosal immunization, especially after aerosol inhalation, so as to prevent the SARS-CoV-2 infection. The vaccine can produce preferably uniform particles between 3 and 10 µm, which can be inhaled through the nasal or oral cavity to reach the lungs, thereby producing a protective immune response to the entire respiratory tract, as well as the lungs, enhancing the effective availability of the vaccine, and enhancing the effectiveness of the vaccine. The vaccine is stable for storage at 2-8 °C, and repeated freeze-thawing does not cause changes in viral activity.

### BRIEF DESCRIPTION OF THE DRAWINGS

Fig. 1 shows an electron micrograph of the recombinant SARS-CoV-2 adenoviral vector.
Fig. 2 shows the results of antibody titers in serum after intramuscular and aerosolized administration of the recombinant SARS-CoV-2 adenoviral vector vaccine.
Fig. 3 shows the results of antibody titers in alveolar lavage fluid after intramuscular and aerosolized administration of the recombinant SARS-CoV-2 adenoviral vector vaccine.
Fig. 4 shows cellular immunity in serum after intramuscular and aerosolized administration of the recombinant SARS-CoV-2 adenoviral vector vaccine.
Fig. 5 shows cellular immunity in alveolar lavage after intramuscular and aerosolized administration of the recombinant SARS-CoV-2 adenoviral vector vaccine.

### DETAILED DESCRIPTION

Unless defined otherwise, all scientific and technical terms used herein have the same meaning as commonly understood by those of ordinary skill in the art to which this invention relates.

The "adenoviral vector vaccine" refers to the vaccine made by recombining the target antigen gene (in the present invention, for example, the S protein gene of SARS-CoV-2) into the adenovirus genome by using the adenovirus as the vector, and using the recombinant adenovirus that can express the antigen gene. Specifically, the gene of SARS-CoV-2 and its various structural proteins can be retrieved through well-known technologies in the art. For example, the gene of SARS-CoV-2 can be shown as GenBank: MT419849.1, and the genes of its various structural proteins: S protein, E protein, M protein can be shown as 21387-25208, 26069-26296, 26347-27015 of GenBank: MT419849.1.

The technical solution of the invention will be described clearly and completely in combination with the embodiments of the invention. Obviously, the described embodiments are only part of the embodiments of the invention, not all of them. Based on the embodiments of the invention, all other embodiments obtained by those of ordinary skill in the art without creative work belong to the scope of the invention.

The human type 5 adenoviral vector and the chimpanzee virus vector belong to the same viral vector vaccine, and the transmucosal drug delivery systems developed by two different vectors have similar effects on immunizing animals. In this embodiment, human type 5 adenoviral vector is taken as an example to explain.

### Embodiment 1: packaging of the recombinant novel coronavirus (SARS-CoV-2) adenoviral vector vaccine

The S antigen gene of SARS-CoV-2 is connected to the shuttle plasmid of the adenovirus, and then the shuttle plasmid co-transfects 293 cells with the skeleton plasmid carrying most of the adenovirus genome. After the shuttle plasmid and the skeleton plasmid co transfect the cells, the Cre/loxP system is used to achieve recombination, thus producing the recombinant adenovirus.

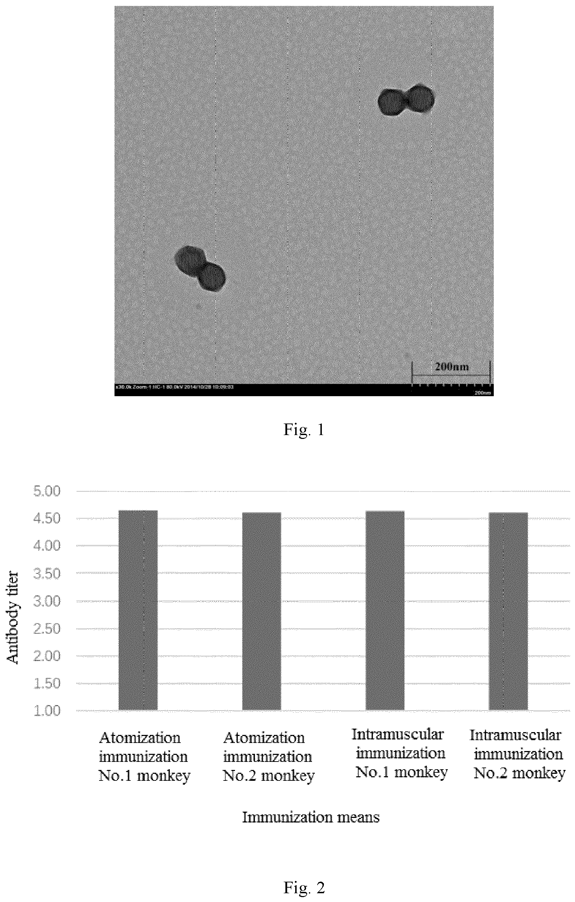

For the successfully packaged recombinant adenovirus, single plaques are serially picked for three times, and those with strong infectivity, high expression and fast assembly speed are selected as the original seeds for the development of production technology and preparation technology. The recombinant adenovirus is observed by an electron microscope, and the result is shown in Fig. 1. As shown in Fig. 1, the typical adenovirus structure is observed under the electron microscope.

### Embodiment 2: preparation study of the recombinant SARS-CoV-2 adenoviral vector vaccine

The liquid preparation formulation of the recombinant SARS-CoV-2 adenoviral vector vaccine is shown in Table 1 and Table 2:

**Table 1 Liquid preparation formulation of recombinant SARS-CoV-2 adenoviral vector vaccine**

| Formulation | Content |
|---|---|
| Recombinant adenovirus (preparation of Embodiment 1) | 1×10¹¹VP/ml |
| Sucrose (mg/ml) | 25 |
| Mannitol (mg/ml) | 50 |
| Sodium chloride (mM) | 50 |
| HEPES (mM) | 2.5 |
| Magnesium chloride (mM) | 2 |
| Tween 80 (mg/ml) | 0.1 |
| Gelatin (mg/ml) | 10 |

**Table 2 Liquid preparation formulation of recombinant SARS-CoV-2 adenoviral vector vaccine**

| Formulation | Content |
|---|---|
| Recombinant adenovirus (preparation of Embodiment 1) | 1×10¹¹VP/ml |
| Sucrose (mg/ml) | 25 |
| Mannitol (mg/ml) | 50 |
| Sodium chloride (mM) | 50 |
| Glycerin (mg/ml) | 1.5 |
| HEPES (mM) | 2.5 |
| Magnesium chloride (mM) | 2 |
| Tween 80 (mg/ml) | 0.1 |
| Hi s(mM) | 5 |
| EDTA(mM) | 0.1 |
| HSA | 0.60% |
| Gelatin (mg/ml) | 10 |

The changes of VP and IFU of the liquid preparation of the recombinant SARS-CoV-2 adenoviral vector vaccine in Table 2 are determined after multiple doses of atomization. The results are shown in Table 3. The results show that the recombinant SARS-CoV-2 adenoviral vector vaccine has good stability, and its specific activity does not change significantly after repeated atomization.

**Table 3 Specific activity changes of recombinant SARS-CoV-2 adenoviral vector vaccine after aerosol administration**

| Name | IFU/mL, × 10^9 | VP number/mL, × 10^11 | Specific activity% |
|---|---|---|---|
| Before spraying | 14.5 | 2.69 | 5.39 |
| After one time of atomization - 1 | 12.5 | 2.01 | 6.22 |
| After two times of atomization - 2 | 12.0 | 2.33 | 5.15 |
| After three times of atomization - 3 | 11.0 | 1.71 | 6.43 |
| After four times of | 11.3 | 2.00 | 5.65 |
| atomization - 4 | | | |

The particle size range of the aerosolized spray of the recombinant SARS-CoV-2 adenoviral vector vaccine in the formulation of Table 2 above is determined and the result shows that the particle size is 1-6 µm with an average of 4 µm.

The atomization time of the recombinant SARS-CoV-2 adenoviral vector vaccine with different administration volumes is measured for three consecutive times, and the results are shown in Table 4. The results show that the liquid preparation of the recombinant SARS-CoV-2 adenoviral vector vaccine is suitable for atomization, and the CV variation of different atomization time is not more than 10%.

**Table 4 Atomization rate of recombinant SARS-CoV-2 adenoviral vector vaccine with different volumes**

| Volume (µ L) | Atomization time 1 (S) | Atomization time 2 (S) | Atomization time 3 (S) | Average value (S) | CV |
|---|---|---|---|---|---|
| 200 | 26 | 27 | 24 | 26 | 5.88 |
| 300 | 32 | 32 | 32 | 32 | 0.00 |
| 400 | 42 | 44 | 43 | 43 | 2.33 |
| 500 | 54 | 53 | 54 | 54 | 1.07 |
| 600 | 67 | 68 | 70 | 68 | 2.25 |
| 1000 | 107 | 106 | 106 | 106 | 0.54 |
| 3000 | 304 | 301 | 303 | 303 | 0.50 |

### Embodiment 3: study on immunogenicity of recombinant SARS-CoV-2 adenoviral vector vaccine

According to Table 2, the recombinant SARS-CoV-2 adenoviral vector vaccine with the same preparation formulation contains 1×10¹¹VP per ml and is used to immunize cynomolgus monkeys by intramuscular injection and aerosol inhalation, and determine IgG in serum and IgA in alveolar lavage fluid.
Experimental animal: cynomolgus monkeys, 2 in each group;
Immunization dose: 1 dose, each dose is 0.5ml, containing 5×10¹⁰VP virus particles;
Immunization mode: intramuscular injection and aerosol inhalation, with the same immunization dose;
Single needle immunization is adopted, blood is collected 4 weeks after immunization, the alveolar lavage fluid is collected, and IgG in serum and IgA in alveolar lavage fluid are determined by ELISA.

The detection results of IgG in serum are shown in Fig. 2. The results show that the above recombinant SARS-CoV-2 adenoviral vector vaccine could immunize the cynomolgus monkeys in two means, intramuscular injection and aerosol inhalation. At the same dose, the IgG antibody titers produced in serum are basically the same.

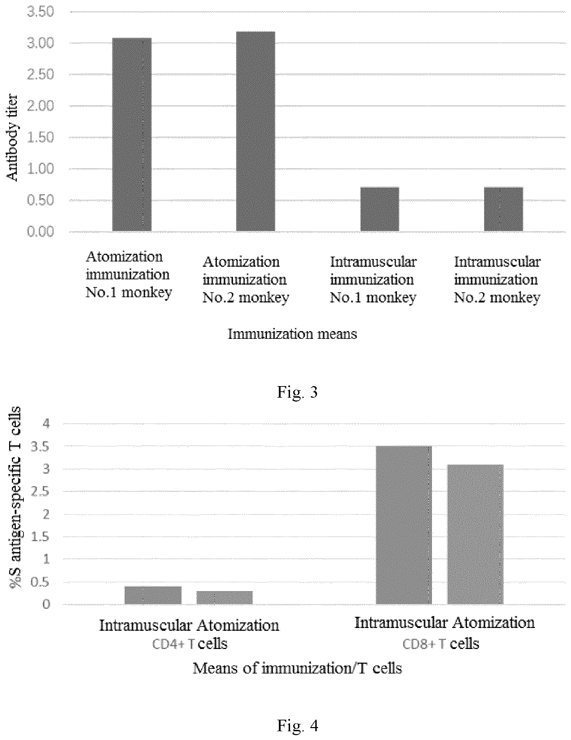

The detection results of IgA in alveolar lavage fluid are shown in Fig. 3. The results show that the above recombinant SARS-CoV-2 adenoviral vector vaccine could immunize the cynomolgus monkeys in two means, intramuscular injection and aerosol inhalation. The muscle immunization do not effectively produce IgA, but the transmucosal immunization of aerosol inhalation realizes detection of high-titer IgA in alveolar lavage.

### Embodiment 4: study on cellular immunity of recombinant SARS-CoV-2 adenoviral vector vaccine

According to Table 2, the recombinant SARS-CoV-2 adenoviral vector vaccine with the same preparation formulation contains 1×10¹¹VP per ml and is used to immunize the cynomolgus monkeys by intramuscular injection and aerosol inhalation, and the cellular immune level in serum and alveolar lavage fluid is measured.
Experimental animal: cynomolgus monkeys, 2 in each group;
Immunization dose: 1 dose, each dose is 0.5ml, containing 5×10¹⁰VP virus particles;
Immunization method: intramuscular injection and aerosol inhalation;
Single needle immunization is adopted, blood is collected 4 weeks after immunization, and cellular immunity is determined.

The detection results of IgG in serum are shown in Fig. 4. The results show that the level of cellular immunity induced by atomized inhalation of the recombinant COVID-19 vaccine is similar to that induced by intramuscular injection.

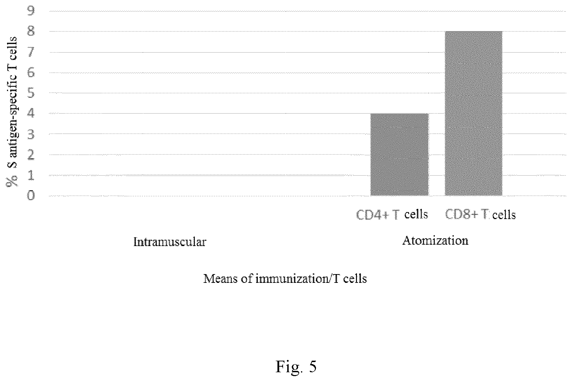

The cellular immunity in the alveolar lavage fluid is measured, and the results are shown in Fig. 5. The results show that the atomized inhalation of the recombinant COVID-19 vaccine could effectively stimulate the cellular immunity level of the alveolar lavage fluid, and the cellular immunity could not be detected in the alveolar lavage fluid of intramuscular injection.

### Embodiment 5: study on protective effect of recombinant SARS-CoV-2 adenoviral vector vaccine

According to Table 2, the recombinant SARS-CoV-2 adenoviral vector vaccine with the same preparation formulation contains 1×10¹¹VP per ml and is used to immunize cynomolgus monkeys by intramuscular injection and aerosol inhalation, and the neutralization antibody titer is determined.
Experimental animal: cynomolgus monkeys, 2 in each group;
Immunization dose: 1 dose, each dose is 0.5ml, containing 5×10¹⁰VP virus particles;
Immunization methods: intramuscular injection and aerosol inhalation;
Single needle immunization is adopted, blood is collected 4 weeks after immunization, and the neutralization antibody titer is determined. The results are shown in the following table:

**Table 5 Neutralizing antibody of recombinant SARS-CoV-2 adenoviral vector vaccine in serum after intramuscular injection and aerosol inhalation**

| | Neutralizing antibody | | Average value |
|---|---|---|---|
| | No.1 monkey | No. 2 monkey | |
| Intramuscular injection | 550 | 580 | 565 |
| Transmucosal immunity - inhalation | 630 | 560 | 595 |

The results show that the above recombinant SARS-CoV-2 adenoviral vector vaccine could produce the neutralizing antibody level in the cynomolgus monkeys immunized with intramuscular injection and aerosol inhalation, and the neutralizing antibody levels produced by the two administration means are similar.

The above is only preferred embodiments of the invention, and does not limit the invention. Any modification, equivalent replacement, etc. made within the spirit and principle of the invention should be included in the protection scope of the invention.

The aforementioned embodiments and methods described in the invention may be different based on the abilities, experiences and preferences of those skilled in the art.

In the invention, only listing the steps of the method in a certain order does not constitute any restriction on the sequence of the steps of the method.

## Claims

1. A SARS-CoV-2 recombinant adenoviral vector vaccine, wherein the vaccine is a transmucosal drug delivery system, and the vaccine comprises an S protein gene of SARS-CoV-2 which is inserted into a recombinant adenoviral vector.

2. The vaccine according to claim 1, wherein the vaccine is a mucosal immune formulation, and the vaccine further comprises pharmaceutically acceptable auxiliary materials, which comprise but are not limited to one or more of a buffer, a protective agent, a stabilizer, a surfactant and an osmotic pressure regulator.

3. The vaccine according to claim 1, wherein the adenoviral vector comprises a human type 5 adenoviral vector or a chimpanzee adenoviral vector;
preferably, the human type 5 adenoviral vector is AdHu2 type or AdHu5 type; and
preferably, the chimpanzee adenoviral vector is selected from AdC6 type, AdC7 type, AdC63 type and AdC68 type.

4. The vaccine according to claim 1, where the transmucosal drug delivery system is selected from: a nasal drop, an aerosol, a spray, a powder spray, a powder, gel, a microsphere agent, a liposome, a membrane, and a suspension; and preferably, the transmucosal drug delivery system is a spray.

5. The vaccine according to claim 1, wherein the recombinant adenovirus further comprises other structural protein genes of SARS-CoV-2;
preferably, the recombinant adenovirus comprises an S protein gene and an M protein gene of SARS-CoV-2; or,
the recombinant adenovirus comprises an S protein gene and an E protein gene of SARS-CoV-2; or,
the recombinant adenovirus comprises an S protein gene, an M protein gene and an E protein gene of SARS-CoV-2.

6. The vaccine according to claim 2, wherein the vaccine comprises: a recombinant adenoviral vector, sucrose, mannitol, sodium chloride, HEPES, magnesium chloride, Tween 80 and gelatin.

7. The vaccine according to claim 2, wherein the vaccine comprises: a recombinant adenoviral vector vaccine, sucrose, mannitol, sodium chloride, glycerin, HEPES, magnesium chloride, Tween 80, His, EDTA, human serum albumin and gelatin.

8. The vaccine according to claim 6, wherein the vaccine is a liquid dosage form, comprising: 1×10⁹~5×10¹¹VP/ml of a recombinant adenovirus, 10-50mg/ml of sucrose, 15-75mg/ml of mannitol, 40-60mM of sodium chloride, 1-5mM of HEPES, 1-SmM of magnesium chloride, 0.05-0.5mg/ml of Tween 80, and 5-15mg/ml of gelatin.

9. The vaccine according to claim 7, wherein the vaccine is a liquid dosage form, comprising: 1×10⁹~5×10¹¹VP/ml of a recombinant adenovirus, 10-50mg/ml of sucrose, 15-75mg/ml of mannitol, 40-60mM of sodium chloride, 0.5-5mg/ml of glycerin, 1-5mm of HEPES, 1-5mm of magnesium chloride, 0.05-0.5mg/ml of Tween 80, 3-7mM of His, 0.05-0.5mM of EDTA, 0.1%-1% of HSA, and 5-15mg/ml of gelatin.

10. The vaccine according to claim 6, wherein the vaccine is a liquid dosage form, comprising: 1×10¹¹VP/ml of a recombinant adenovirus, 25mg/ml of sucrose, 50mg/ml of mannitol, 50mM of sodium chloride, 2.5 mM of HEPES, 2mM of magnesium chloride, 0.1mg/ml of Tween 80, and 10mg/ml of gelatin.

11. The vaccine according to claim 7, wherein the vaccine is a dosage form, comprising:1×10¹¹VP/ml of a recombinant adenovirus, 25mg/ml of sucrose, 50mg/ml of mannitol, 50mM of sodium chloride, 1.5mg/ml of glycerin, 2.5 mM of HEPES, 2mM of magnesium chloride, 0.1mg/ml of Tween 80, 5mM of His, 0.1mM of EDTA, 0.60% of HSA, and 10mg/ml of gelatin.

12. The vaccine according to claim 1, wherein the vaccine is a freeze-dried preparation.

13. Use of an S protein gene of SARS-CoV-2 in preparation of a vaccine to prevent a SARS-CoV-2 infection, wherein the vaccine is a transmucosal drug delivery system.

14. Use of a recombinant adenovirus in preparation of a vaccine to prevent a SARS-CoV-2 infection, wherein the recombinant adenovirus is inserted with an S protein gene of SARS-CoV-2, and the vaccine is a transmucosal drug delivery system.

15. The use according to claim 13 or 14, wherein the transmucosal drug delivery system is selected from: a nasal drop, an aerosol, a spray, a powder spray, a powder, gel, a microsphere agent, a liposome, a membrane, and a suspension.

16. The use according to claim 13 or 14, wherein the transmucosal drug delivery system is a spray.
